# EUROPEAN PATENT APPLICATION

(11) **EP 3 666 265 A1**
(43) Date of publication of application: **17.06.2020**
(21) Application number: 20150592.2
(22) Date of filing: 22.05.2013
(51) Int. Cl.: A61K 31/00

(54) **IMPROVED COMPLEXES AND COMPOSITIONS CONTAINING CURCUMIN**

(30) Priority: 22.05.2012 NZ 12600146
(62) Divisional of application: 13793432.9
(71) Applicant: Cave, Harold Gordon, Hamilton 3204 (NZ)
(72) Inventor: Cave, Harold Gordon, Hamilton 3204 (NZ)
(74) Representative: Whitfield, Gillian Janette

(57) **Abstract**

The present invention relates to a complex including a phospholipid and curcumin, characterised in that some or all of the phospholipid is a marine phospholipid. The present invention further relates to a composition including such a complex and a method of preparing such a complex.

## Description

### TECHNICAL FIELD

The present invention relates to improvements in and relating to the bioavailability of curcumin, and methods of producing complexes and compositions providing improved bioavailability of curcumin.

### BACKGROUND ART

Curcumin is a compound present in the spice turmeric. Curcumin has been shown in many studies to have pharmacologic effects such as antioxidant, anti-inflammatory, antiproliferative and antiangiogenic activities. As such, curcumin represents a target to fight diseases such as cancer, heart disease diabetes, Crohn's disease and various neurological diseases. For this reason, there has been significant research on curcumin.

A significant advantage of curcumin is its wide acceptance by the general public due to it being a natural compound used for centuries as a spice in food such as curries. A further advantage is that, even at high doses, there are little or no side effects. It is also relatively cheap to source, and stores well at room temperature.

Despite these advantages, an overriding issue which has yet to be addressed is curcumin's well known problem of low bioavailability in animals. This is thought to be due to a combination of factors including its poor solubility and hence poor absorption, rapid elimination from the animal's digestive system and/or through rapid metabolism.

In the past, this poor solubility has been overcome, at least in *in vitro* studies, by adding carriers such as DMSO or Tween 80 which help to increase the solubility of curcumin. However, addition of these carriers in a therapeutic medicament would not be suitable, primarily because carriers such as DMSO lead to a foul taste, adds to the manufacturing cost and process, and detract from the advantage that curcumin is a natural product (which many consumers prefer).

Another solution used to substantially raise the dosage of curcumin, with the hope that some of it will be absorbed. However, using this approach, there is little control over how much is absorbed if any, and/or there could be potential issues with excessive loading of curcumin having a negative effect on the person.

Combining curcumin with an oil can improve the uptake of curcumin systemically. However, because curcumin does not bind with oils, the majority drops out of suspension quickly after mixing.

Agitating the curcumin and oil mixture vigorously can provide a slightly improved product due to a small percentage of the curcumin being solubilised upon such agitation. Regardless of how vigorously the curcumin and oil mixture is mixed, centrifuging the product will effectively precipitate most of the curcumin from the oil.

Numerous approaches have been taken to alleviate the above absorption/stability issues and to maximise from the beneficial effects of curcumin.

These include preparation of liposomal or phospholipid structures, nanoparticles, and structural analogues. Anand et al., Mol. Pharmaceutics, 2007, 4 (6), 807-818 provides a good review of these different approaches.

WO 2007/101551 describes a phospholipid complex with curcumin using phospholipids from vegetable or synthetic origin. A molar ratio of curcumin to lipid of 1;2 or 1;4 was provided having about 16.9% curcumin in the resulting complex. However, the resulting product was a viscous wax. This would make it substantially impossible to encapsulate in soft gels, and hence the product would need to be provided in a tablet form. Although tablets are a suitable form for delivery of the complex, from the manufacturing perspective, encapsulation can be a more attractive option particularly for oil based formulations. Encapsulation in soft gels is only readily achievable if the resulting complex solution is not too viscous. An alternative method is using hard gel capsules whereby the paste is pumped into the gelatin capsules at slightly elevated temperatures.

A further option is to combine curcumin with adjuvants. Compounds like piperine, quercetin and / or Omega-3 polyunsaturated fatty acids, such as docosahexaenoic acid (DHA) and/or eiscosapentaenoic acid (EPA) have recently been shown to produce a synergistic therapeutic effect when used in combination with curcumin, although the exact modes of action are still uncertain. These approaches have also been outlined in Anand *et al*, 2007.

It is also thought that this synergy of curcumin is limited to a relatively small subset (about 8) of Omega 3 polyunsaturated fatty acids (including DHA and EPA). For example, Altenburg et al., BMC Cancer 2011, 11;149 describes the synergy of DHA and curcumin in inhibiting numerous breast cancer cell lines.

In a further study, Swamy et al., Nutrition and Cancer 2008, 60: S1, 81-89 reported that a ratio of about 2.5:1 (DHA to curcumin) showed the greatest effect on apoptosis in BxPC-3 cells, a form of pancreatic cancer cells.

Although there is reasonable information guiding the formulator to optimal ratios of curcumin to adjuvants such as DHA, it can often be difficult to formulate a composition retaining the desired ratios (which may differ depending on the exact therapeutic effect desired), whilst also trying to accommodate issues around instability, insolubility and poor absorption of curcumin, and rheological factors of the composition such as its preferred viscosity requirements and form of administration.

Therefore, despite certain advances, there is still significant need to improve on the bioavailability and therapeutic effect of curcumin, the ability to increase loading of curcumin, the stability of resulting compositions, and the ease/cost of manufacturing the medicament in a convenient dosage form.

It is an object of the present invention to address one or more of the foregoing problems or at least to provide the public with a useful choice.

All references, including any patents or patent applications cited in this specification are hereby incorporated by reference. No admission is made that any reference constitutes prior art. The discussion of the references states what their authors assert, and the applicants reserve the right to challenge the accuracy and pertinency of the cited documents. It will be clearly understood that, although a number of prior art publications are referred to herein, this reference does not constitute an admission that any of these documents form part of the common general knowledge in the art, in New Zealand or in any other country.

Throughout this specification, the word "comprise", or variations thereof such as "comprises" or "comprising", will be understood to imply the inclusion of a stated element, integer or step, or group of elements integers or steps, but not the exclusion of any other element, integer or step, or group of elements, integers or steps.

Further aspects and advantages of the present invention will become apparent from the ensuing description which is given by way of example only.

### DISCLOSURE OF THE INVENTION

According to a first aspect of the present invention there is provided a complex including a phospholipid and curcumin,
characterised in that some or all of the phospholipid is a marine phospholipid.

According to a further aspect of the present invention there is provided a composition including a complex with a phospholipid and curcumin,
characterised in that some or all of the phospholipid in the complex is a marine phospholipid.

According to a further aspect of the present invention there is provided a method of preparing a complex or composition substantially as herein described above the method including the steps of:
a) forming a first solution by dissolving a quantity of curcumin in a solvent;
b) forming a further solution by adding slowly to the first solution a suitable quantity of a marine phospholipid
c) processing the further solution to form the complex
d) separating the complex from the solvent by distillation.

According to a further aspect of the present invention there is provided a method of treatment using the composition substantially as herein described, wherein the composition is used to treat or prevent, or at least to provide complementary treatment or prevention, to one or more of the following conditions:
- cancer,
- heart disease,
- diabetes,
- Crohn's disease, and
- Neurological disease.

The present invention surprisingly and advantageously benefits from the use of marine based organisms that have a high content of phospholipids and are naturally enriched with polyunsaturated fatty acids such as DHA and EPA. In brief, the present invention:
- allows formation of a stable complex using a marine phospholipid, and/or by forming a stable complex, prevents separation of the components (therefore the present invention helps to improve effective absorption of the curcumin); and/or
- the marine source provides a high level of phospholipids, and by default a high level of polyunsaturated fatty acids such as DHA and EPA (inherently present in the tails of the marine phospholipids). This increased level of fatty acids helps to achieve a desired molar ratio of curcumin to fatty acids, which in turn provides the synergistic effects seen between the fatty acids and the curcumin.

### Marine phospholipid

Throughout this specification the term "marine phospholipid" should be taken as meaning any phospholipid which is sourced from sea-based organisms including, without limitation, fish and shellfish wherein the phospholipid or phospholipids contains at least one type of polyunsaturated fatty acid, and most preferably either DHA and/or EPA.

Some specific examples of such marine phospholipids are from marine oils include mussel oil, krill oil, salmon oil, squid oil and so forth. Another example of marine oils may be the oil from the roe of fish or shellfish.

As also highlighted in other documents such as EP 1871399, marine phospholipids (typically sourced from marine oils) that are also rich in these polyunsaturated fatty acids include algae, phytoplankton and other plant based organisms. These marine phospholipid sources are clearly encompassed by the present invention.

Marine oils such as these exemplified above beneficially have relatively high phospholipid content. Therefore these may be beneficially used for the present invention to help achieve a preferred molar ratio of curcumin to phospholipids and fatty acids, as discussed further below. By using marine oils such as those exemplified above, the complex also beneficially provides the synergistic therapeutic effect offered by the Omega -3 fatty acids such as DHA or EPA with the curcumin.

It should be appreciated that it is possible that the phospholipids are first extracted from a marine oil (for instance through acetone precipitation) and then subsequently bound to the curcumin to form the complex. During the extraction process, a waxy substance may result after the above acetone precipitation of the phospholipids, which may then be thinned down with a diluent before being complexed, or bound, with the curcumin.

Alternatively the phospholipids may be obtained by adding several volumes of chilled ethanol to a suitable oil such as krill oil in which case the phosphatidylcholine (a preferred type of phospholipid as discussed further below) will become solubilised in the supernatant and can be drawn off and obtained in that manner.

Throughout this specification the term complex should be generally understood to be the binding together, through some form of chemical interaction or forces, of at least one part of the phospholipid and at least one curcumin compound. Without wishing to be bound by theory, the inventor envisages that the binding occurs through binding of the curcumin to the choline head group of the phospholipid so as to form a complex.

Preferably, the composition includes marine oil. This is a preferred option because it is thought that other components of the marine oil (besides the phospholipid) may improve the therapeutic effectiveness or stability of curcumin. Due to their number and range (for example, mussel oil has about 91 different types of fatty acids) the precise nature and effect of those other components cannot be explained in further detail herein.

Despite this synergy between the fatty acids DHA and/or EPA with curcumin being known for considerable time, it is not known to complex the curcumin to a marine derived phospholipid(s) to form a complex to achieve the beneficial effects discussed herein. Instead, researchers have only provided co-therapy of omega 3 with curcumin, but never have complexed these key components.

It is instead known, and widely taught in the prior art, to complex vegetable oil such as soyabean lecithin oil with curcumin. Such soy phospholipids are known to be highly absorbed in humans, and do not show any chronic effects on animals *in vivo,* even at high dosages and hence present themselves as ideal candidates for complexing. Also the high amounts of polyunsaturated fatty acids like linoleic acid present in soyabean oil has made it an attractive option for reducing the risk of diseases like heart disease. Yet, a major disadvantage is that there is no reported synergy with shorter chain fatty acids in vegetable oils (like linoleic acid) and curcumin. Also, plant and land animal based phospholipids do not provide the same plethora or health effects which are provided by marine phospholipids.

### Phospholipid

Throughout this specification the term "phospholipid" should be taken as meaning any type of lipid that includes a hydrophobic tail and hydrophilic head. Phospholipids, in context of this invention, are used to form a complex whereby the choline group (if using phosphatidylcholine) head binds with the curcumin, while the phosphatidyl portion envelops the bound part.

Preferably, the marine oil contains greater than 20% w/w phospholipid. Marine oils such as salmon oil, mussel oil, krill oil and squid oil are all known to have phospholipid contents above 20%. For example, marine oils with particularly high levels of phospholipid are mussel oil and krill oil with levels up to 65% w/w phospholipid.

Most preferably, the marine oil contains approximately 40% w/w phospholipid.

More preferably, the phosphdolipid is selected from the group consisting of phosphatidylcholine (PC), phosphatidic acid (PA), phosphatidylethanolamine (PE), phosphatidylserine (PS), phosphatidylinositol (PI), phosphatidylinositiol phosphate (PIP), phosphatidylinositiol biphosphate (PIP2), and phosphatidylinositiol triphosphate (PIP3).

Most preferably, at least a portion of the phospholipid in the complex is phosphatidylcholine (PC).

PC accounts for at least 50% of the outer membrane of all the trillions of cells in the human body. It is possible that the choice of PC as the marine phospholipid, in light of their prevalence in the cells of the human body, may help to improve absorption of the curcumin and fatty acids into cells where they can perform their therapeutic roles and synergy.

However, potentially any one or combination of phospholipids could be used with the present invention.

Examples of marine oils that have both PC and PE include mussel oil, krill oil and salmon oil.

Alternatively, the phospholipid is selected from the broad class of phosphosphingolipids.

In this alternative embodiment, the phospholipid is selected from the group consisting of ceramide phosphorylcholine or ceramide phosphorylethanolamine (Sphingomyelin, SPH or Cer-PE respectively) and ceramide phospholipid.

### Curcumin

Throughout this specification the term curcumin should be taken as meaning any curcuminoid. A curcuminoid may simply be curcumin as shown in the structure below, or may be a derivative of curcumin with varied chemical groups which provide improved stability or other pharmacokinetic properties of the compound as is known in the art.

The curcumin may be isolated from a natural source such as turmeric, or it may be synthetically prepared through a range of techniques known in the art.

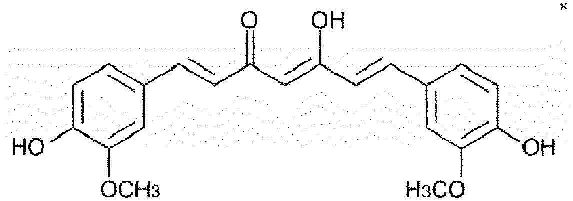

In some situations to treat particular treatments such as prostate cancer, demethoxycurcumin may be used. In Cuomo et al, J. Nat. Prod. 2011, 74 664-669, it was shown that phospholipid formulation increased the absorption of demethoxylated curcuminoids much more than that of curcumin, therefore making demethoxycurcumin particularly applicable for use in the current invention. A further commonly used alternative curcumin is bisdemethoxycurcumin.

However, other forms of curcumin, may be used with the present invention without departing from the scope thereof.

Preferably, the complex includes above 1% w/w curcumin to phospholipid.

Preferably, the complex includes between 1 to 55% w/w curcumin to phospholipid.

As discussed previously, a higher concentration and loading of curcumin is one strategy to improve the ultimate aim of greater absorption of curcumin into the body, yet using the present invention the complex allows the improved absorption of the curcumin so the additional concentration of curcumin may be better utilised.

More preferably, the complex includes approximately between 20-45% w/w curcumin to phospholipid. Those skilled in the art will appreciate that it is possible to increase the level of curcumin beyond 8% within the complex of the present invention. The means to do so is discussed further in the specification.

### Molar ratios

Preferably, the molar ratio of the curcumin to phospholipids in the complex is in the range of about 1:1 to 1:20.

Preferably, the molar ratio of the curcumin to phospholipids in the complex is in the range of about 1.1 to 1:5.

As discussed previously with reference to *Altenburg* and *Swomy,* there are reports to show that the most preferred molar ratio which provides the most effect synergy for the cancer cell lines studied was about 2.5 to 1 (DHA to curcumin). However, the inventor believes that a ratio upwards of 20:1 (DHA and/or other fatty acids such as EPA to curcumin) also will show a synergy, albeit to a lesser degree. As will be apparent to those skilled in the art, depending on the particular therapeutic use (for example, the type of cancer cells to be targeted), the molar ratio may need to be varied to provide a heightened synergy.

Such preferred ratios were seen to effectively be complexed with the trial examples performed by the inventor and are well documented to provide suitable complexing of curcumin with the phospholipid. For example, WO 2007/101551 shows (in Example 4) that up to 16% w/w curcumin could be achieved in a stable complex when the ratio of curcumin to phospholipid is as high as 1:4. Obviously, one could also achieve a stable complex if the amount of phospholipid is increased in this ratio.

An example of how a particularly preferred 1:16 ratio of curcumin to phospholipids can be achieved is provided below.

Mussel oil has approximately 65% w/w phospholipids, and also contains about 24% w/w EPA, 13% DHA and about 4-5% of six other Omega 3 acids, totalling about 41-42% Omega 3 polyunsaturated fatty acids which are predominantly bound to the phospholipids. Therefore, if one were to add 4 kg of curcumin to 100 kg (approximately 100 L) of mussel oil, a ratio of approximately 1:16 (curcumin to phospholipid) may be achieved in the resulting complex, and provide about a 40 g/L (or 4% w/w) curcumin in the complex.

Research shows that there can be a therapeutic effect of curcumin in the body (suggested by *in vitro* trials) with a molar ratio of curcumin to phospholipids of only 10 uM.

### Adjuvants

Preferably, additional components may be incorporated into the composition. These components do not necessarily bind to the complex but may help to improve stability, or for instance, may be added as adjuvants to improve the therapeutic effect of the composition.

Preferably, the composition includes quercetin which may bond with and form a complex with phospholipids.

Preferably, the composition includes piperine.

Both quercetin and piperine are known to be valuable adjuvants to curcumin, for example to increase absorption or potency of the curcumin for an improved therapeutic effect.

Preferably, some or all of the adjuvants are simultaneously complexed with the phospholipids. As discussed below, the quercetin and piperine are preferably added together with the curcumin prior to dissolving in the solvent, and prior to the addition of the phospholipids sourced from the marine oil. In this way, the quercetin is able to be complexed with the phospholipids in the same manner as the curcumin.

### Method of manufacture

According to a further aspect of the present invention there is provided a method of preparing a composition substantially as described above, the method includes the steps of:
a) dissolving a quantity of curcumin in ethanol to form a first solution;
b) slowly adding a marine phospholipid to the first solution to form a second solution, and
c) boiling off the ethanol from the second solution to result in a complex formed between the phospholipid and curcumin.

In a particularly preferred embodiment, the method includes the steps of:
a) dissolving a quantity of curcumin in a solvent (preferably warm) to form a first solution, placing said solution under vacuum and 40-50 degrees centigrade until gentle refluxing;
b) slowly adding a marine phospholipid the first solution to form a second solution;
c) processing the second solution to form the complex; and
d) separating the complex from the solvent, preferably by vacuum distillation.

### Solvent

The solvent(s) used in the present invention may vary depending upon the type or amount of curcumin used, and the type or amount of phospholipid and/or other components intended for the composition or complex. Therefore the exact composition of the solvent should not be seen as limiting.

Preferred embodiments in which the first constituent is a natural plant or animal based extract may utilise one or more solvents from the following list. It should be appreciated however that this list is not exhaustive and therefore should not be seen as limiting.
- Hexane
- Benzene
- Toluene
- Diethyl ether
- Chloroform
- Acetic acid
- Butanol
- Isopropanol
- Propanol
- Ethanol
- Methanol
- Formic acid
- Dimethyl Sulfoxide,
- Acetone.

Preferably, the solvent is a protic solvent. Throughout this specification, the term "protic solvent" should be taken as meaning any solvent that has a hydrogen atom bound to an oxygen (i.e. a hydroxyl group) or a nitrogen (i.e. an amine group). From the list above, protic solvents include acetic acid, butanol, isopropanol, ethanol, methanol and formic acid.

Most preferably, the solvent is ethanol.

Preferably step a) includes mixing approximately 40-50 parts volume of solvent to about 1 part curcumin.

This ratio helps to ensure the curcumin is properly dissolved and prevents precipitation during the mixing process. The process may also be aided by performing the dissolving step at warmer temperatures.

Solvents such as ethanol may be advantageous as utilise food grade quality ethanol is commercially available for processing techniques such as this.

The step of processing the second solution to form the complex may be achieved through numerous ways. There are many known techniques to form complexes using phospholipids and a drug or compound, for example as documented in WO 2007/101551. Yet without forming the complex, the curcumin and phospholipid are unstable and will separate quickly. This is not only a problem for shelf-life stability, but it also lowers the bioavailability of the curcumin as noted above. To improve absorption, techniques including forming complexes help to keep the curcumin bound to the phospholipids for improved absorption in the body.

Preferably, step c) includes separation by way of evaporation.

Preferably, step c) includes heating the second solution to raise the temperature of the second solution to greater than the boiling point of the solvent, but less than the remaining components in the second solution.

Preferably, step c) includes heating the second solution in a pressure vessel.

Most preferably, the second solution is heated at below atmospheric pressure. This reduces the boiling point of the solvent and fluid and allows efficient evaporation at lower temperatures. For example, the method may be performed under vacuum.

In some embodiments the pressure in the pressure vessel may be reduced sufficiently to facilitate evaporation of the solvent at room temperature.

In some embodiments the pressure in the pressure vessel may be raised to increase the boiling point of the solvent and fluid.

In some preferred embodiments the step of boiling may be performed at atmospheric pressure or above, whereby the further solution is heated in order to exceed the boiling point of the ethanol.

In preferred embodiments the ethanol that is boiled off is condensed and recovered.

In some preferred embodiments the step of boiling may be performed by reducing the pressure in a vessel containing the further solution, the reduced pressure lowering the effective boiling point of the ethanol.

Reducing the temperature of evaporation by applying a vacuum is advantageous as it reduces the chance of the oil becoming rancid. Oils are sensitive to heat, light and exposure to oxygen. The use of a vacuum reduces both the contribution of heat and of oxygen to the degeneration of the oil.

Preferably, step c) includes applying a slow vacuum at around 40-50°C for 1 - 2 hours and then a full vacuum until all the ethanol is boiled off.

In some embodiments a combination of reduced pressure and heating of the further solution may be employed to evaporate off the ethanol.

In some embodiments the pressure may be increased, thereby increasing the boiling point of the ethanol, thereby requiring a greater degree of heating to evaporate the ethanol.

It will be appreciated that the rate of evaporation may be controlled by increasing and decreasing the pressure within the pressure vessel in which the further solution is contained.

In preferred embodiments the vacuum pressure in the pressure vessel is in the range of 1-100 torr.

### Advantages of the Present Invention

- Bonding the curcumin to a marine phospholipid improves bioavailability as the curcumin is protected by the complex.
- Bonding the curcumin to the marine phospholipid prevents separation which is seen when curcumin is simply added in combination with oil (without complexing).
- Sourcing the phospholipid from a marine organism allows a preferred molar ratio of curcumin to omega 3 in the complex to be achieved.
- Using a marine oil sourced phospholipid beneficially maintains the synergistic effect seen between Omega 3 fatty acids with curcumin. Omega 3 fatty acids are by default always present in marine phospholipids.
- There is good public acceptance and trust of marine oils used in therapeutic compositions.
- Marine phospholipids also provide a plethora of other health benefits which are not provided by plant or land animal based phospholipids.
- In marine phospholipids, the omega 3s are bound primarily to the phospholipids rather than triglycerides, so it is relatively easy to concentrate the phospholipids, which by default substantially increases the concentration of omega 3s and at the same time removes the triglycerides.
- The present invention also provides provision of a stable complex and ability to achieve higher molar ratios of curcumin to fatty acids by increasing the concentration of curcumin and phospholipid. However, in such embodiments, the most preferred option would be tabletting or use of hard gels due to the higher viscosity of the composition.

### BRIEF DESCRIPTION OF DRAWINGS

Further aspects of the present invention will become apparent from the following description which is given by way of example only and with reference to the accompanying drawings in which:

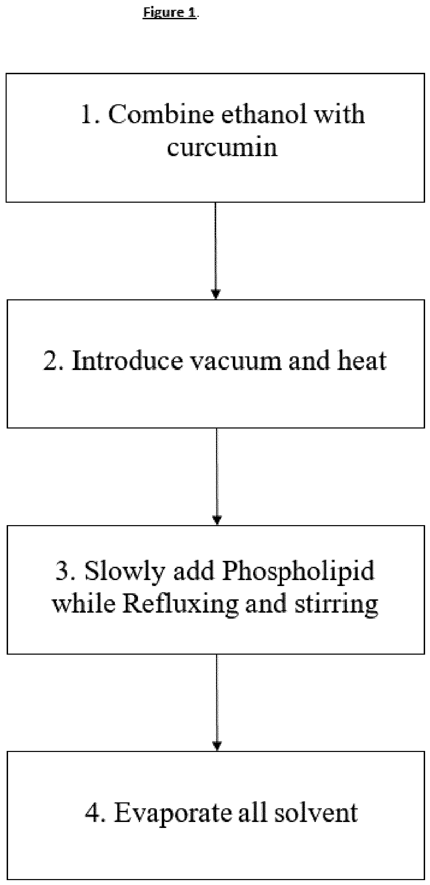

- Figure 1: is a flow diagram of one preferred method of preparing a composition in accordance with the present invention.

### BEST MODES FOR CARRYING OUT THE INVENTION

Figure 1 is a flow diagram illustrating the overall stages of producing a particularly preferred composition in accordance with the present invention. Box 1 of Figure 1 depicts the stage of combining of the various components such as curcumin, ethanol, marine phospholipids. It will be appreciated that the combination may be performed in any order without departing from the scope of the invention; however the preferred option is adding the phospholipid to the curcumin and ethanol.

Box 2 of Figure 1 depicts a mixing step in which the further solution is mixed in order to evenly distribute the different components. It will be appreciated that this step could be combined with the combination step of box 1 and/or the reflux/evaporation of step 3. The mixing step 2 may be performed for a fixed period prior to reflux/evaporation step 3 so as to ensure even mixing.

Box 3 of Figure 1 depicts a reflux/evaporation step. Reflux/evaporation may be achieved in a number of ways, including:
- boiling the mixed further solution at ambient pressure until the solvent has been evaporated off;
- boiling the mixed further solution at elevated temperatures at above ambient pressure;
- boiling the mixed further solution at reduced temperatures at below ambient pressure.

The boiling point of the solvent may be substantially lower than that of other fluids in the further fluid, as such the temperature/pressure may be controlled so that only the solvent is boiled off.

The evaporation stage may be controlled either manually, or by way of a control system, to control the rate of evaporation as is known in the art.

Typically the curcumin to be used will be initially a powder. The powder is dissolved in the solvent and the phospholipid will be added slowly to the first solution. If the one or more further compounds include powdered compounds these can also be dissolved in the solvent prior to combining with the other compounds. If dilution is required, a diluent such as olive oil may be added.

In some embodiments a further step may be included which involves taking a sample of fluid during the reflux/evaporation stage and centrifuging the sample to ascertain the degree of bonding between at least the first constituent and at least one of the at least one further constituents. It will be appreciated the in cases where bonding has not occurred centrifuging results in sedimentation of the first constituent forming. Where strong bonding has occurred little or no sedimentation is formed during centrifuging.

### Examples

The invention is further described by way of reference to the following examples. These examples should not however be construed as limiting.

### Example 1 - Method of manufacture

In this example, the amounts of each component added is based on a "by weight" amount as illustrated in Example 2.
Step 1: In a stirred vacuum tank, 40-50 parts of warm ethanol is mixed with an amount of curcumin, quercetin and piperine.
Step 2: Mixing occurs under reflux and vacuum (less than 100 mBar) and at a temperature of approximately 40-50°C until all the components are in suspension.
Step 3: An amount of krill oil is slowly added to the solution formed by step 2.
Step 4: After 30 minutes of reflux, the mixture from step 3 is further mixed under a slow vacuum at around 40-50°C for 1- 2 hours and then a full vacuum until all the ethanol is boiled off and collected in a evaporator leaving the curcumin bound to the phospholipid as a complex.

### Example 2 - Exemplary master composition mix of the present invention

| Component | Amount |
|---|---|
| curcumin C3 | 9.4 kg |
| krill oil (50% phospholipids) | 90 kg |
| Ethanol (to be removed) | 500 kg |
| Piperine | 0.1 kg |
| Quercetin | 0.5 kg |
| Total | 100 kg (after ethanol removed) |

### Example 3

To illustrate the stability of the curcumin-phospholipid complex, the following study was performed by the inventor.

A control composition was produced by directly mixing 4% by weight curcumin powder with 500ml of krill oil with a blender. The mixture was vigorously mixed in the blender for 20 minutes.

A comparable trial composition (4% curcumin) was then produced according to the method outlined in example 1 (method of manufacture) and example 2 (example composition) and in accordance with the present invention.

The control and trial compositions were both placed in an IEC DPR6000 centrifuge and subjected to 4500 rpm (5550 G) for 1 hour. The resulting precipitate was weighed for each of the control mixtures.

The results showed that the control composition had only 0.3% by weight as soluble curcumin. This correlates to known rates of between 0.2 to 0.5% w/w solubilisation of curcumin. Oppositely, the trial composition did not produce any precipitate indicating that the entire 4% w/w of curcumin had been bound to the krill oil derived phospholipids.

Aspects of the present invention have been described by way of example only and it should be appreciated that modifications and additions may be made thereto without departing from the scope thereof.

## Claims

1. A complex including a phospholipid and curcumin,
**characterised in that** some or all of the phospholipid is a marine phospholipid.

2. The complex as claimed in claim 1, wherein the phospholipid includes DHA and/or EPA.

3. The complex as claimed in claim 1 or 2, wherein at least a portion of the phospholipid in the complex includes phosphatidylcholine.

4. The complex as claimed in any one of the preceding claims, wherein an amount of phospholipids in the complex are sourced from lecithin.

5. The complex as claimed in any one of the preceding claims, wherein the complex also includes piperine and/or quercetin.

6. A composition including the complex as claimed in any one of the preceding claims.

7. The composition as claimed in claim 7, wherein the composition includes a marine oil.

8. The composition as claimed in claim 6 or 7, wherein the composition includes an additional source of polyunsaturated fatty acids DHA and/or EPA.

9. The composition as claimed in any one of claims 6 to 8, wherein the composition includes an adjuvant.

10. The composition as claimed in any one of claims 6 to 9, wherein the composition includes quercetin and/or piperine.

11. The composition as claimed in any one of claims 6 to 10, wherein the composition is provided in a liquid or semi-liquid form in a soft gel capsule.

12. A method of preparing a complex as claimed in any one of claims 1 to 5, the method including the steps of:
a. dissolving a quantity of curcumin in a solvent to form a first solution;
b. mixing the first solution with a quantity of a marine phospholipid to form a second solution;
c. processing the second solution to form the complex; and
d. separating the complex from the solvent.
